# EUROPÄISCHE PATENTSCHRIFT

(11) **EP 4 368 154 B1**
(45) Veröffentlichungstag und Bekanntmachung des Hinweises auf die Patenterteilung: **25.03.2026**
(21) Anmeldenummer: 22020550.4
(22) Anmeldetag: 14.11.2022
(51) Int. Cl.: A61F 5/02

(54) **RÜCKENORTHESE**
BACK ORTHOSIS
ORTHÈSE DORSALE

(43) Veröffentlichungstag der Anmeldung: 15.05.2024
(73) Patentinhaber: Sassu, Antonio, 09011 Calasetta (SU) (IT)
(72) Erfinder: Sassu, Antonio, 09011 Calasetta (SU) (IT)
(74) Vertreter: Schneider, Andreas

(56) Entgegenhaltungen:
- EP-A1- 3 488 828
- EP-A1- 3 536 285
- DE-A1- 102017 108 839
- JP-A- 2019 196 567

## Beschreibung

Die Erfindung betrifft eine Rückenorthese, mit einem an einem Oberkörper eines Patienten abnehmbar anbringbaren Stützelement, und mit einer Gurtanordnung, die ausgebildet ist, in einer Trageposition der Rückenorthese das Stützelement körpernah an dem Rücken des Patienten zu fixieren.

Derartige Rückenorthesen sind in verschiedenen Ausführungen bekannt. Oftmals sind diese Orthesen jedoch kompliziert aufgebaut und bestehen aus einer Vielzahl von zusammenwirkenden Teilen. Sie sind daher nicht nur teuer in der Herstellung, sondern lassen sich meist auch nur mühevoll anlegen.

Beispiele für solche Rückenorthesen sind in EP 3 488 828 A1, die den Oberbegriff des Anspruchs 1 offenbart, DE 10 2017 108839 A1 und EP 3 536 285 A1 beschrieben. Über die genannten Nachteile hinaus üben diese Rückenorthesen aufgrund ihres konstruktiven Aufbaus stets nur an einer Stelle des Rückens punktuell Druck auf die Wirbelsäule aus und sind daher für eine Stützung des Rückens in seiner Gesamtheit nicht geeignet. Eine Aufgabe der vorliegenden Erfindung ist es daher, eine Rückenorthese bereitzustellen, die besonders einfach aufgebaut ist und aus wenigen Teilen besteht, so daß sie preiswert in der Herstellung ist und sich einfach anlegen läßt.

Diese Aufgabe wird durch eine Rückenorthese nach Anspruch 1 gelöst. Vorteilhafte Ausführungen der Erfindung sind in den Unteransprüchen angegeben.

Erfindungsgemäß umfaßt die Rückenorthese für den Rücken eines Patienten ein an einem Oberkörper eines Patienten abnehmbar anbringbares Stützelement sowie eine Gurtanordnung, die ausgebildet ist, in einer Trageposition der Rückenorthese das Stützelement körpernah an dem Rücken des Patienten zu fixieren, wobei das Stützelement strangartig ausgebildet ist und in der Trageposition eine Längserstreckung im Wesentlichen entlang der Wirbelsäule des Patienten aufweist, wobei das Stützelement entlang seiner Längserstreckung gekrümmt ist, insbesondere eine Anzahl geschwungener Bereiche aufweist, insbesondere derart, daß einer oder mehrere dieser geschwungenen Bereiche an die menschliche Anatomie der Wirbelsäule angepaßt sind, und wobei das Stützelement einen oder mehrere elastisch flexible Beugeabschnitte aufweist, welche Beugeabschnitte eine reversible, zumindest abschnittsweise Beugung des Stützelements quer zu seiner Längserstreckung ermöglichen, wobei die Gurtanordnung einen mit dem unteren Ende des Stützelements verbundenen Hüftgurt umfaßt, der ausgebildet ist, in der Trageposition das Stützelement an einer ersten Stelle des Rückens des Patienten festzulegen, wobei sie den Oberkörper des Patienten vollumfänglich umschlingt, wobei die Gurtanordnung einen mit einem mittleren und/oder einem oberen Teil des Stützelements verbundenen Brustgurt umfaßt, der ausgebildet ist, in der Trageposition das Stützelement an einer zweiten Stelle des Rückens des Patienten festzulegen, welche zweite Stelle von der ersten Stelle beabstandet ist, wobei wenigstens einer der geschwungenen Bereiche des Stützelements, nämlich ein an dem oberen Ende des Stützelements angeordneter oder das obere Ende des Stützelements bildender geschwungener Bereich, einen Beugeabschnitt aufweist oder einen Beugeabschnitt bildet, welcher Beugeabschnitt in seinem nicht vorgespannten Zustand nicht an dem Rücken des Patienten anliegt, sondern von dem Rücken absteht, und in der Trageposition durch die Gurtanordnung vorgespannt ist, zum Zweck einer dauerhaften Haltungskorrektur des Patienten durch eine Zugwirkung auf die Wirbelsäule nach hinten.

Mit anderen Worten ist das Stützelement zumindest nicht vollständig starr bzw. biegesteif, sondern wenigstens abschnittsweise elastisch verformbar ausgebildet.

Im angelegten Zustand der Orthese ist das Stützelement bei aufrechtem Oberkörper des Patienten, d.h. bei einem sitzenden oder stehenden Patienten, vertikal angeordnet.

Vorzugsweise ist das Stützelement im wesentlichen S-förmig ausgeführt, d.h. es zeigt eine zweifache Biegung. Als besonders vorteilhaft hat sich eine im wesentlichen Doppel-S-förmige Ausführung erwiesen, bei der diejenige Biegung, welche den Abschluß des ersten S-förmigen Abschnitts bildet, zugleich diejenige Biegung ist, welche den Anfang des zweiten S-förmigen Abschnitts bildet.

Vorzugsweise ist die genaue Form der S-Kurve (n) des Stützelements patientenindividuell ausgebildet, d.h. insbesondere derart, daß einer oder mehrere der geschwungenen Bereiche des Stützelements an die Anatomie der Wirbelsäule des Patienten angepaßt sind.

Vorzugsweise ist das Stützelement in seiner konstruktiven Ausführung, insbesondere hinsichtlich seiner Länge, Breite, Flexibilität, der Ausführung der Biegungen etc., patientenindividuell anfertigbar und/oder anpaßbar.

Es gibt im einfachsten Fall lediglich drei Grundkomponenten der Orthese, nämlich das Stützelement und die Gurtanordnung, die einen Hüftgurt und einen Brustgurt umfaßt, so daß die Orthese sehr einfach aufgebaut ist. Zusätzliche Stützplatten, Pelotten oder dergleichen sind nicht erforderlich. Die Gurte können mehrteilig ausgebildet sein und Gurtelemente aufweisen, die über Befestigungsmittel in Form von Klettverschlüssen oder dergleichen aneinander befestigbar sind. Der Brustgurt ist vorzugsweise nicht mit dem Hüftgurt verbunden. Vorzugsweise lassen sich Hüftgurt und Brustgurt unabhängig voneinander anlegen, was das Herstellen der Trageposition der Orthese erleichtert.

Der Hüftgurt ist vorzugsweise nach Art einer um die Hüfte bzw. Taille anlegbaren Lumbalbandage ausgebildet. Der Hüftgurt ist vorzugsweise lösbar mit dem Stützelement, insbesondere mit dem unteren Ende des Stützelements, verbunden. Der Hüftgurt ist erfindungsgemäß ausgebildet, in der Trageposition der Orthese das Stützelement an einer ersten Stelle des Rückens des Patienten festzulegen, wobei sie den Oberkörper des Patienten vorzugsweise vollumfänglich umschlingt. Vorzugsweise weist der Hüftgurt eine in der Trageposition am Rücken des Patienten angeordnete taschenartige Aufnahme auf, in welcher das Stützelement mit seinem unteren (lumbosakralen) Ende in dem Hüftgurt festlegbar ist. Vorzugsweise weist der Hüftgurt im vorderen bzw. bauchseitigen Bereich einen Verschluß auf. Über den Verschluß läßt sich der Hüftgurt beim Anlegen öffnen.

Der Brustgurt ist vorzugsweise lösbar mit dem Stützelement verbunden, insbesondere mit einem mittleren und/oder oberen Teil des Stützelements. Der Brustgurt ist erfindungsgemäß ausgebildet, in der Trageposition das Stützelement an einer zweiten Stelle des Rückens des Patienten festzulegen, welche zweite Stelle von der ersten Stelle beabstandet ist. Mit der Festlegung des Stützelements an zwei voneinander beabstandeten Stellen am Rücken des Patienten wird die Möglichkeit geschaffen, mit Hilfe der Gurtanordnung das Stützelement elastisch zu verformen. Ein Verformen des Stützelements des entsprechend ausgebildeten Stützelements kann mit anderen Worten mittels eines der Grundkomponenten der Orthese erfolgen, ohne daß ein zusätzliches Spannelement oder dergleichen benötigt wird.

Vorzugsweise erstreckt sich das Stützelement in der Trageposition über einen wesentlichen Teil der Wirbelsäule des Patienten, so daß die Rückenorthese gezielt auf die Wirbelsäule des Patienten einwirken kann. Vorzugsweise erstreckt sich das Stützelement über die gesamte Länge der Wirbelsäule. Alternativ hierzu erstreckt sich das Stützelement nur entlang eines Teils der Wirbelsäule, wodurch eine gezielte Unterstützung der Wirbelsäule in einem zu unterstützenden oder zu therapierenden Bereich ohne eine eventuell unerwünschte Beeinflussung eines anderen Wirbelsäulenbereiches ermöglicht wird.

Vorzugsweise ist das Stützelement schienen- oder leistenförmig ausgeführt, insbesondere als flacher und damit flächig an dem Rücken des Patienten anlegbarer Profilstab mit einem Mehrkant-, vorzugsweise Vierkant-Querschnitt. Eine Bauart des Stützelements nach Art einer flachen Rückenschiene ermöglicht es, daß das Stützelement im angelegten Zustand der Orthese wirbelsäulennah auf dem Rücken aufliegt. Die Breite des Stützelements ist dabei signifikant kleiner als dessen Länge. Vorzugsweise ist die Breite des Stützelements über dessen gesamte Länge unverändert.

Vorzugsweise ist das Stützelement einteilig ausgeführt, wodurch es nicht nur besonders robust sondern auch besonders einfach herstellbar ist. Alternativ besteht das Stützelement aus mehreren, miteinander in Verbindung stehenden Stützelementteilen, vorzugsweise derart, daß die Teile derart variabel aneinander festlegbar sind, daß die Größe des Stützelements, insbesondere seine Länge, variabel einstellbar ist.

Vorzugsweise besteht das Stützelement aus einem Epoxidharz-Glasfaser-Verbundwerkstoff oder einem glasfaserverstärkten Kunststoff. Auf diese Weise wird eine besonders leichte Konstruktion bei hoher Stabilität gewährleistet. Anstelle der Glasfaser-Komponente können auch andere geeignete Materialien verwendet werden, insbesondere Kohlefaser, Aramidfasern, Kevlar^{®} oder dergleichen. Durch die Verwendung dieser Materialien ist die Orthese vergleichsweise leicht und stellt auch bei längerem Tragen keine Belastung für den Patienten dar. Das Gesamtgewicht der Orthese beträgt vorzugsweise weniger als 500 Gramm.

Das Stützelement ist vorzugsweise mehrschichtig aufgebaut, wobei das verwendete Material mit Hilfe von geeigneten Formwerkzeugen in die gewünschte Form gepreßt wird. Durch die Wahl der Anzahl der Schichten können die Materialeigenschaften des Stützelements entlang seiner Längserstreckung ortsgenau definiert werden. Dies betrifft insbesondere die Elastizität bzw. Flexibilität des Stützelements.

Erfindungsgemäß wirkt die Orthese auf den Rücken und die Schultern des Patienten und unterstützt die Aufrichtung der Haltung, indem
wenigstens einer der geschwungenen Bereiche des Stützelements, insbesondere ein an dem oberen Ende des Stützelements angeordneter oder das obere Ende des Stützelements bildender geschwungener Bereich, einen Beugeabschnitt aufweist oder einen Beugeabschnitt bildet, wobei dieser Beugeabschnitt als Federelement dient und in der Trageposition, d.h. im angelegten Zustand der Orthese, durch die Gurtanordnung vorgespannt ist. Das Vorspannen des Beugeabschnitts, das je nach Ausführung des Stützelements auch ein Vorspannen des gesamten Stützelements zur Folge haben kann, erfolgt vorzugsweise durch einen dann als Spanngurt wirkenden Teil des Brustgurtes, der in diesem Fall vorzugsweise als kombinierter Brust-/Schulter-Gurt ausgeführt ist, oder durch einen als Spanngurt wirkenden, mit dem Brustgurt verbundenen Schultergurt. Das Vorspannen des Beugeabschnitts erfolgt zum Zweck einer dauerhaften Haltungskorrektur des Patienten durch eine Zugwirkung auf die Wirbelsäule, genauer eine Zugwirkung auf den Oberkörper bzw. den Schulterbereich des Patienten nach hinten. Zum Vorspannen des Beugebereiches und damit zur Aufrichtung der Wirbelsäule dienen vorzugsweise als Teile der Gurtanordnung ausgeführte Schultergurte, die im angelegten Zustand im Schulter-Achsel-Bereich des Patienten plaziert sind und als Reklinationsgurte wirken. Durch die Wirkung von zwei über die Schultergurte übertragenen Kräften und einer dazwischenliegenden entgegengesetzten, durch das Stützelement aufgebrachten dritten Kraft kann der Oberkörper und damit die Wirbelsäule aufgerichtet werden. Auf diese Weise ergibt sich entlang der Längserstreckung des Stützelements, d.h. entlang einer vertikalen Linie entlang der Wirbelsäule, ein bestimmter Abstand zwischen der Wirbelsäule einerseits und der Rückenstütze andererseits. In Abhängigkeit von der Größe dieses jeweiligen Abstandes wird die Wirbelsäule in jeder Vertikalposition mehr oder weniger nach hinten gezogen.

Vorzugsweise dient die elastische Funktion des Stützelements zur Unterstützung antagonistischer Muskeln des Patienten, indem zum Zweck eines kraftunterstützten Aufrichtens eines nach vorn geneigten Oberkörpers des Patienten der wenigstens eine Beugeabschnitt des Stützelements bei einer nach vorn orientierten Neigung des Oberkörpers des Patienten über den Grad der Vorspannung hinaus weiter spannbar ist derart, daß das Stützelement ein auf den Oberkörper wirkendes, entgegen der nach vorn orientierten Neigung rückstellendes Aufrichtmoment auf den Oberkörper ausübt.

Das bedeutet, daß die Orthese nicht nur "statisch", also in einem Ruhezustand des Oberkörpers des Patienten, z.B. im Stehen oder im Sitzen angewendet werden kann, sondern auch "dynamisch", d.h. in der Bewegung bzw. während der Bewegung des Oberkörpers. Wird nämlich bei einer Bewegung des Oberkörpers nach vorn das Stützelement elastisch verformt, indem es nach vorn verbogen wird, unterstützt das Stützelement bei der Rückbewegung, also einem Aufrichten des Oberkörpers aus der nach vorn gebeugten Stellung, den Oberkörper, indem es ihn in die aufrechte Position zurückzieht. Anders ausgedrückt unterstützt das Stützelement ein Aufrichten des Oberkörpers, was insbesondere bei kranken oder älteren Personen beschwerlich sein kann. Üblicherweise ist es für diese Personen nicht das Problem, sich nach vorn zu beugen, sondern sich wieder aufzurichten, wobei die Orthese hilft. Das Stützelement dient bei diesem Vorgang als Speicher für potentielle Energie während der Hin-Bewegung. Beim Aufrichten wird die potentielle Energie wieder zurückgegeben. Die durch die antagonistischen Muskeln aufgebrachte Aufrichtkraft wird durch die in die gleiche Richtung wirkende Federkraft des Stützelements unterstützt. Die Rück-Bewegung erfolgt anders ausgedrückt unterstützt mit einer geringeren Belastung der Muskeln und des Skeletts. Dadurch, daß die Kräfteübertragung teilweise über das Stützelement, also ein aus Sicht des Skeletts externes Element erfolgt, wird das Skelett weniger belastet. Insgesamt ergibt sich somit ein zusätzlicher dynamischer Effekt der Orthese. Durch die Orthese wird in diesen Fällen auch die Stärke der Kompression der Wirbelsäule verringert.

Vorzugsweise weist das Stützelement wenigstens einen Stützabschnitt auf, der in der Trageposition zumindest zeitweise, nämlich in Abhängigkeit von der Neigung des Oberkörpers, vorzugsweise jedoch dauerhaft - den Rücken abstützt, indem es einen Vertikalanteil der Gewichtskraft des Oberkörpers des Patienten aufnimmt. Der wenigstens eine Stützabschnitt ist vorzugsweise starr bzw. biegesteif oder im wesentlichen biegesteif. Er ist vorzugsweise zwischen dem oberen Ende und dem unteren Ende des Stützelements angeordnet. Speziell in einem statischen Gleichgewichtszustand lastet dann ein Vertikalanteil des Gewichts des Oberkörpers auf dem Stützelement. Auf diese Weise wird der Patient beim Tragen seines Gewichts entlastet. Die Unterstützung des Oberkörpers durch das Stützelement erfolgt dabei vorzugsweise ausschließlich in einer definierten Richtung, nämlich nach hinten. In Abhängig von der Art und Weise der Krümmung des Stützelements kann mehr oder weniger Gewicht des Patienten übernommen bzw. aufgefangen werden. Mit anderen Worten übernimmt das Stützelement dann einen Teil der Funktion der Wirbelsäule, nämlich eine teilweise Tragfunktion des Gewichts des Patienten. Die Orthese wirkt in dieser Situation wie eine zusätzliche, externe Wirbelsäule, d.h. nach Art eines "Bypass". Während normalerweise das Gewicht des Oberkörpers, einschließlich des Gewichtes des Kopfes, die Wirbelsäule in einer Art und Weise deformiert, die für die Haltung des Patienten ungünstig ist, wird eine solche Belastung durch die unterstützende Wirkung der Orthese teilweise kompensiert, so daß die Wirbelsäule in einer bevorzugten Lage verbleiben kann. Das Stützelement ist dabei vorzugsweise derart gestaltet, daß es auf eine gewichtsbedingte Belastung der Wirbelsäule automatisch und selbsttätig mit einer entlastenden Gegenreaktion, nämlich der Abstützung des Oberkörpers, "antwortet". Eine horizontal wirkende Gegenkraft der Rückenstütze kompensiert die durch die Wirbelsäule ausgeübte Druckkraft und bewirkt, daß sich die Wirbelsäule weniger stark krümmt. In diesen Fällen wird also nicht nur die Wirbelsäule nach hinten gezogen. Die Wirbelsäule wird auch durch Orthese unterstützt bzw. gestützt. Ist der Rücken des Patienten gekrümmt, wird ein Teil des Körpergewichts von dem Stützelement aufgenommen und von der Orthese getragen. Der Rücken wird entlastet, da die Wirbelsäule nicht das gesamte Gewicht tragen muß. Die Muskeln, die für das Tragen des Oberkörpers benötigt werden, werden ebenfalls weniger belastet.

Vorzugsweise ist das Stützelement torsionsweich ausgebildet, um eine torsionale Flexibilität des Stützelements zu erhalten. Das Stützelement bewirkt somit eine Zugwirkung auf die Wirbelsäule nach hinten, ohne eine Torsion des Oberkörpers zu verhindern oder übermäßig zu behindern. Die vorliegende Tordierbarkeit führt mit anderen Worten zur Gewährleistung bzw. möglichst geringen Einschränkung der Oberkörperbeweglichkeit des Patienten, auch bei angelegter Orthese. Trotzdem bei einem Verdrehen des Oberkörpers auch eine Kraft auf das Stützelement wirkt, ist das resultierende Torsionsmoment jedoch gering, so daß der Oberkörper nur minimal beeinflußt wird. Unterstützend hilft hierbei, daß das Stützelement vorzugsweise ein in Zugrichtung, d.h. nach hinten, flaches Bauteil ist, so daß das Stützelement in diese Bewegungsrichtung starr bzw. biegesteif ist, während es in Torsionsrichtung flexibel bleibt.

Die Erfindung bezweckt, mit Hilfe eines anatomisch geformten, vertikalen Stützelements mit vordefinierter Flexibilität einen gesunden Stützdruck auf die gesamte Wirbelsäule des Patienten auszuüben und auf diese Weise die durch die Schwerkraft ausgeübte Druckbelastung gleichmäßig auf die Wirbelsäule und das Stützelement selbst zu verteilen, was zu einer deutlichen Linderung von Schmerzen und Muskel-Skelett-Belastungen führt.

Mit der Erfindung wird eine ergonomisch-anatomische Rückenstütze geschaffen. Die Orthese ist besonders einfach aufgebaut und besteht aus wenigen Teilen, so daß sie preiswert in der Herstellung ist. Zugleich läßt sich die Orthese einfach anlegen, sie ist intuitiv nutzbar und wirkungsvoll zur biomechanischen Entlastung des Oberkörpers.

Die erfindungsgemäße Orthese eignet sich insbesondere zur ergänzenden Unterstützung bei der posttraumatischen und posturalen Rehabilitation. Eine primäre Funktionalität der Orthese liegt dabei in der Rehabilitation und Umerziehung zur Unterstützung eines therapeutischen Weges, insbesondere im Rahmen einer Rehabilitationstherapie. Eine sekundäre Funktionalität der Orthese liegt in der Haltungskorrektur und der Unterstützung von muskuloskelettalen Belastungen.

Die erfindungsgemäße Orthese findet Anwendung bei Störungen unterschiedlicher Art, wie Hexenschuß, Knochentraumata, Muskelzerrungen und postoperative Schäden, bei denen die normale Funktionsfähigkeit der Muskeln und der Wirbelsäule nicht vollständig gegeben ist.

Die erfindungsgemäße Orthese wirkt in erheblichem Maße rehabilitierend und unterstützend auf die Wirbelsäule, da ihre Haupteigenschaft darin besteht, die Belastung des Bewegungsapparates durch Fehlhaltungen zu verringern, insbesondere aufgrund von Schmerzsyndromen bei Stürzen oder Operationswegen die eine lange Rekonvaleszenz erfordern. Die Orthese findet vorteilhafte Anwendung u.a. bei Skoliose, Schmerzen im Lendenwirbelbereich aufgrund von Skelettbelastungen und/oder Muskelkontrakturen sowie andere Problemen, auch solchen, die lediglich mit der täglichen Aktivität zusammenhängen, wie langem Stehen, das schmerzhafte Kompressionen der Wirbelsäule verursacht, oder besonders anstrengenden Arbeiten.

Durch die Ausübung eines gewissen Drucks entlang der Wirbelsäule stützt die Orthese die Wirbelsäule, indem sie sie in der richtigen Position hält und so Druckbelastungen und Schmerzsyndrome, die durch die Schwerkraft oder andere Faktoren verursacht werden, deutlich verringert. Zur Verringerung der Belastung des Patienten trägt auch bei, daß die Orthese derart ausführbar ist, daß sie ein vergleichsweise geringes Gewicht aufweist, vergleichsweise unauffällig ist und darüber hinaus besonders einfach und bequem tragbar ist.

Die erfindungsgemäße Orthese wird während eines ausreichend langen Zeitraumes vorzugsweise dauerhaft getragen. Dabei gibt die Orthese, ohne die Muskeln zu belasten, dem Körper Zeit, sich zu rehabilitieren und die volle Funktion wiederzuerlangen, ohne die Muskeln und Gelenke zu belasten. Die Orthese kann problemlos auch über einen längeren Zeitraum getragen werden, beispielsweise mehrere ganze Tage.

Zusammenfassend beruht die Erfindung u.a. auf folgenden Überlegungen. In Bezug auf die Muskelstruktur des menschlichen Körpers, die in Agonisten- und Antagonisten-Muskeln unterteilt ist, ist bekannt, daß ein stehender Körper das Resultat der gleichzeitigen Wirkung dieser beiden Muskeltypen ist, die auf die Skelettstruktur reagieren und zu einem Gleichgewicht der wirkenden Kräfte führen, so daß jedem Agonisten-Muskel ein Antagonisten-Muskel entgegensteht. Die Bewegungen der Gliedmaßen, des Rumpfes und aller beweglichen Körperteile sind das Ergebnis eines momentanen Ungleichgewichts der Kräfte, die auf ein Element des Körperskeletts einwirken, und zwar nach dem Prinzip Kraft = Masse x Beschleunigung, wobei im ersten Teil einer Bewegung die agonistischen Kräfte überwiegen, und um den Körper wieder in seine ursprüngliche Position zu bringen, die anderen Kräfte, die antagonistischen, überwiegen. Es ist auch bekannt, daß bei den Rückenmuskeln die Bewegungsrichtungen nach unten und nach oben verlaufen, so daß die agonistische Arbeit teilweise durch die Schwerkraft ausgeführt wird und somit durch die vertikale Haltung mit einem Gewicht, dem Kopf, auf der Spitze, entlastet wird. In diesem Fall sind es die antagonistischen Muskeln, die besonders beansprucht und benachteiligt werden. Die muskulär-vertebralen Probleme machen sich besonders bemerkbar, wenn eine Person versucht, sich aus der Rückenlage in eine aufrechte Haltung zu begeben. In diesem Kontext stellt die erfindungsgemäße Orthese eine wirksame Hilfe insbesondere für die antagonistischen Muskeln dar. Bei einer Bewegung nach unten müssen die Muskeln beim Beugen des Rückens die elastische Kraft des Stützelements der Orthese überwinden und leisten mehr Arbeit, aber diese Arbeit wird als potentielle Energie in dem gebogenen vertikalen Stützelement so lange gespeichert, bis sie bei der Rückbewegung freigesetzt wird und auf diese Weise die Belastung des antagonistischen Muskels verringert wird. Mit Hilfe dieser Lösung wird erreicht, daß die agonistischen Muskeln die antagonistischen Muskeln unterstützen, wobei die ersteren verstärkt und die letzteren unterstützt werden. Auf diese Weise werden die Kräfte auf das Stützelement übertragen, die Wirbelsäule wird bei beiden Bewegungen entlastet und teilweise überbrückt.

Ein Ausführungsbeispiel der Erfindung wird nachfolgend anhand der Zeichnungen näher erläutert. Hierbei zeigen:
- Fig. 1: die Orthese im ihrer Trageposition (Rückenansicht des Patienten),
- Fig. 2: die Orthese im ihrer Trageposition (Brustansicht des Patienten),
- Fig. 3: das Stützelement (Draufsicht),
- Fig. 4: das Stützelement (Seitenansicht),
- Fig. 5: eine schematische Darstellung des Stützelements mit betonter Doppel-S-Krümmung (Seitenansicht),
- Fig. 6: eine Prinzipskizze der statischen Situation vor dem Anlegen der Orthese (Seitenansicht),
- Fig. 7: eine Prinzipskizze der statischen Situation nach dem Anlegen der Orthese (Seitenansicht),
- Fig. 8: eine Prinzipskizze der statischen Situation nach dem Anlegen der Orthese (Draufsicht),
- Fig. 9: eine Prinzipskizze der dynamischen Situation nach dem Anlegen der Orthese bei einer Neigebewegung des Oberkörpers nach vorn (Seitenansicht),
- Fig. 10: eine Prinzipskizze der statischen Situation nach dem Anlegen der Orthese mit Darstellung der Stützfunktion (Seitenansicht).

Sämtliche Figuren zeigen die Erfindung nicht maßstabsgerecht, dabei lediglich schematisch und nur mit ihren wesentlichen Bestandteilen. Gleiche Bezugszeichen entsprechen dabei Elementen gleicher oder vergleichbarer Funktion.

"Vorn" oder "vorderes" bedeutet dabei, das ein Bauteil im angelegten Zustand der Orthese, d.h. in der Trageposition, in Bezug auf den Körper des Patienten vorn, d.h. am Bauch des Patienten, angeordnet ist bzw. bezieht sich auf ein sich in diese Richtung erstreckendes bzw. in diese Richtung weisendes Bauteil, während "hinten" oder "hinteres" bedeutet, das ein Bauteil im angelegten Zustand der Orthese, d.h. in der Trageposition, in Bezug auf den Körper des Patienten hinten, d.h. am Rücken des Patienten, angeordnet ist bzw. bezieht sich auf ein sich in diese Richtung erstreckendes bzw. in diese Richtung weisendes Bauteil. Die Angaben "oben" bzw. "unten" beziehen sich ebenfalls auf den bestimmungsgemäßen Verwendungszustand, d.h. die Trageposition der Orthese.

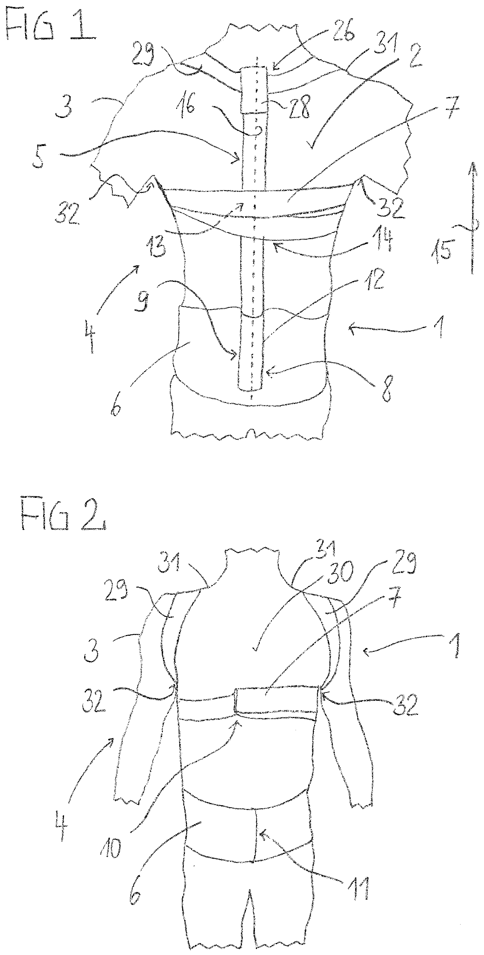

Die Rückenorthese 1 für den Rücken 2 eines Patienten 3 umfaßt ein an dem Oberkörper 4 des Patienten 3 abnehmbar anbringbares Stützelement 5 und eine Gurtanordnung, die ausgebildet ist, in einer Trageposition der Rückenorthese 1 das Stützelement 5 körpernah an dem Rücken 2 des Patienten 3 zu fixieren, siehe Fig. 1 und 2.

Die Gurtanordnung umfaßt einen Hüftgurt 6 und einen Brustgurt 7. Diese können mehrteilig ausgebildet sein. Die Gurtanordnung weist jedoch zumindest zwei Gurtelemente auf.

Der Hüftgurt 6 ist lösbar mit dem Stützelement 5, nämlich mit dem unteren (lumbosakralen) Ende 8 des Stützelements 5, verbunden.

Der Hüftgurt 6 ist nach Art einer um die Hüfte bzw. Taille des Patienten 3 anlegbaren Lumbalbandage ausgebildet. Der Hüftgurt 6 ist ausgebildet, in der Trageposition das Stützelement 5 an einer ersten Stelle 9 des Rückens 2 des Patienten 3 festzulegen, wobei sie den Oberkörper 4 des Patienten 3 vollumfänglich umschlingt bzw. umschließt. Der Hüftgurt 6 weist im vorderen bzw. bauchseitigen Bereich einen Verschluß 11 auf. Der Hüftgurt läßt sich über den Verschluß 11 beim Anlegen öffnen. Das Stützelement 5 ist mit seinem unteren Ende 8 in einer taschenartigen Aufnahme 12 festlegbar, die im hinteren bzw. rückenseitigen Bereich des Hüftgurts 6 vorgesehen ist.

Der Brustgurt 7 ist lösbar mit dem Stützelement 5, nämlich mit einem mittleren Teil 13 des Stützelements 5, verbunden, indem es in der Trageposition das Stützelement 5 an einer zweiten Stelle 14 des Rückens 2 des Patienten 3 festlegt, welche zweite Stelle 14 von der ersten Stelle 9 beabstandet ist. Der Brustgurt 7 ist nicht mit dem Hüftgurt 6 verbunden.

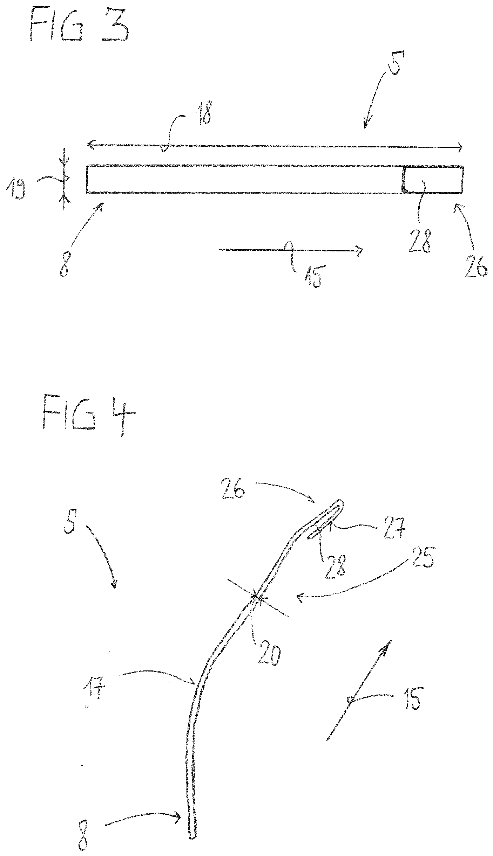

Das Stützelement 5 ist strangartig ausgebildet und weist in der Trageposition eine Längserstreckung 15 im Wesentlichen entlang der (in Fig. 1 mit durchbrochener Linie dargestellten) Wirbelsäule 16 des Patienten 3 auf. Das Stützelement 5 erstreckt sich in der Trageposition über einen wesentlichen Teil der Wirbelsäule 16 des Patienten 3, insbesondere über deren gesamte oder die im wesentlichen gesamte Länge der Wirbelsäule 16. Das Stützelement 5 ist schienen- oder leistenförmig ausgeführt, nämlich als flacher, an dem Rücken 2 des Patienten 3 anlegbarer Profilstab mit einem Vierkant-Querschnitt. Dabei ist das Stützelement 5 einteilig ausgeführt, siehe Fig. 3 und 4.

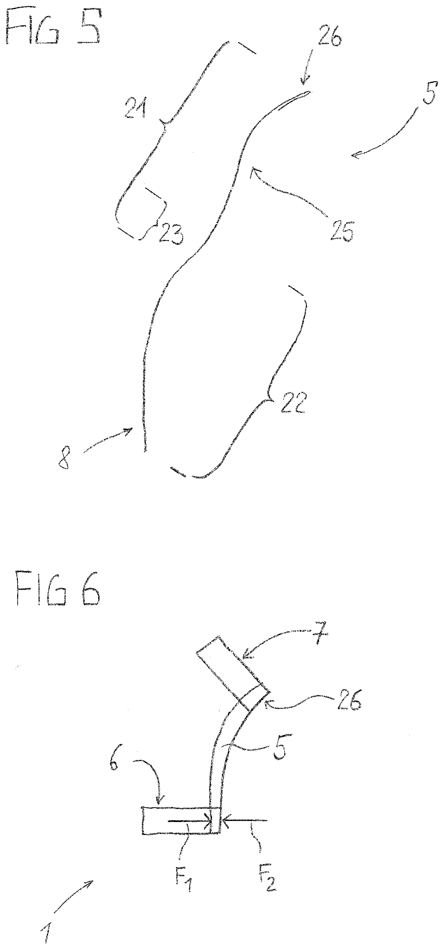

Das Stützelement 5 ist entlang seiner Längserstreckung 15 gekrümmt bzw. gebogen. Es weist eine Anzahl geschwungener Bereiche 17 auf, die an die menschliche Anatomie der Wirbelsäule angepaßt sind. Das Stützelement 5 weist eine im wesentlichen Doppel-S-förmige Ausprägung auf, bei der diejenige Biegung 23, welche den Abschluß des ersten S-förmigen Abschnitts 21 bildet, zugleich diejenige Biegung ist, welche den Anfang des zweiten S-förmigen Abschnitts 22 bildet, siehe Fig. 5.

Die genaue Form der S-Kurve(n) des Stützelements 5 ist patientenindividuell ausgebildet derart, daß einer oder mehrere der geschwungenen Bereiche 17 des Stützelements 5 an die Anatomie der Wirbelsäule 16 des Patienten 3 angepaßt sind. Außerdem sind auch die Länge 18 und die Breite 19 des Stützelements 5 patientenindividuell angepaßt. Die für eine Person mit einer Körpergröße von ca. 1,70 bis 1,85 m vorgesehene Länge 18 des Stützelements 5 beträgt ca. 600 mm, bei einer im wesentlichen über die gesamte Längserstreckung 15 gleichbleibenden Breite 19 von ca. 60 mm und einer in diesem Beispiel ebenfalls über die gesamte Längserstreckung 15 gleichbleibenden Dicke 20 von ca. 3 mm.

Das Stützelement 5 besteht aus einem Epoxidharz-Glasfaser-Verbundwerkstoff. Das Gesamtgewicht der Orthese beträgt im Beispiel etwa 450 g. Das Stützelement 5 ist mehrschichtig aufgebaut. Aufgrund des Aufbaus und des verwendeten Materials ist das Stützelement 5 torsionsweich ausgebildet, so daß es auch im angelegten Zustand eine entsprechende Beweglichkeit des Oberkörpers 4 erlaubt.

Das Stützelement 5 weist einen elastisch flexiblen Beugeabschnitt 25 auf, die eine reversible, zumindest abschnittsweise Beugung des Stützelements 5 quer zu seiner Längserstreckung 15 ermöglichen. Anders ausgedrückt ist das Stützelement 5 zumindest nicht vollständig starr bzw. biegesteif, sondern wenigstens abschnittsweise elastisch verformbar ausgebildet.

Im Gebrauchszustand der Orthese 1 ist das Stützelement 5 mit seinem unteren Ende 8 in einer eigens dafür an dem Hüftgurt 6 vorgesehenen Tasche 12 aus unelastischem, verstärktem Gewebe eingesetzt. Die Tasche 12 befindet sich in der Trageposition der Orthese 1 zentral im Bereich der Lendenfaszie des Patienten 3. Beim Festschnallen des Hüftgurtes 6 wird das untere Ende 8 des Stützelements 5 am Rücken 2 des Patienten 3 fixiert. Die Haltekraft am unteren Ende 8 des Stützelements 5 dient lediglich zur sicheren Fixierung. In dem unteren Fixierpunkt 9 herrscht ein Kräftegleichgewicht, wie in Fig. 6 durch die Pfeile F1 und F2 angedeutet. Dieser untere Fixierpunkt 9 bildet den Ausgangspunkt, von dem aus das Stützelement 5 seine Unterstützungsfunktion ausübt.

Das obere Ende 26 des Stützelements 5 ist hakenförmig umgebogen, so daß sich ein Befestigungs- bzw. Haltehaken 27 mit einem schmalen Aufnahmeschlitz 28 für einen Schultergurt 29 ergibt. Der als Teil des Brustgurtes 7 dienende Schultergurt 29, der als ein elastisches Band von etwa 80 mm Breite ausgeführt ist, ist im Gebrauchszustand in den Aufnahmeschlitz 28 eingefädelt derart, daß er in der Lage ist, das Stützelement 5 in Richtung des Rückens 2 zu ziehen. Aufgrund der Hakenform des oberen Endes 26 des Stützelements 5 kann der Schultergurt 29 sich nach oben nicht von dem Stützelement 5 lösen. Das obere Ende 26 des Stützelements 5, genauer gesagt der Boden des Aufnahmeschlitzes 28, bildet somit einen Angriffspunkt für den Schultergurt 29 zur Beaufschlagung des Stützelements 5 mit einer Zugkraft.

In der Trageposition verlaufen die beiden Stränge des Schultergurtes 29 von dem rückseitigen Aufnahmeschlitz 28 kommend beidseitig über die Schultern 31 des Patienten 3 und anschließend unmittelbar unter den beiden Achseln 32 hindurch zurück nach hinten, wo sie, in ihrer Funktion als Brustgurt, auf dem Rücken 2 - im Bereich des mittleren Teils 13 zwischen dem oberen Ende 26 und dem unteren Ende 8 des Stützelements 5 - über das Stützelement 5 hinweggeführt sind und das Stützelement 5 auf diese Weise an der einen oberen Fixierpunkt bildenden zweiten Stelle 14 an dem Rücken 2 festlegen. Die beiden Enden des Gurtes 29 werden über dem Stützelement 5 verschlungen und erneut auf die Vorderseite des Oberkörpers 4 zurückgeführt, wo sie auf der Brust 30 aneinander befestigt sind. Zu diesem Zweck weist auch der Brustgurt 7 einen Verschluß 10 auf, über den sich der Brustgurt 7 beim Anlegen öffnen läßt, beispielsweise mit elastischen Klettverschlüssen für den orthopädischen Einsatz, wodurch zugleich eine Einstellung der Länge des Brustgurtes 7 erfolgen kann.

Der Teil des Stützelements 5, der im angelegten Zustand der Orthese 1 mit dem Oberkörper 4 in Berührung kommt, sowie der als elastisches Band ausgeführte Schultergurt, der über die Schultern 31 verläuft, sind mit einem weichen, antiallergischen Material überzogen, so daß die Funktion der Orthese 1 nicht beeinträchtigt wird und sie auch im Alltag bequem zu benutzen ist.

Nachfolgend werden die Kräfte betrachtet, die auf den Rücken 2 und die Schultern 31 des Patienten 3 wirken und die Aufrichtung der Haltung unterstützen ("statische Betrachtung"), siehe Fig. 6, 7 und 8.

Der das obere Ende des Stützelements 5 bildende geschwungene Bereich des Stützelements 5 bildet einen Beugeabschnitt 25, der als Federelement dient und in der Trageposition durch die beiden Schultergurte 29 vorgespannt ist. Die Schultergurte 29 dienen mit anderen Worten als aufrichtende Reklinationsgurte zum Aufrichten der Wirbelsäule 16. Dieser Beugeabschnitt 25 liegt in seinem nicht vorgespannten Zustand nicht an dem Rücken 2 des Patienten 3 an, sondern steht von dem Rücken 2 ab, d.h. das obere Ende 26 des Stützelements 5 weist vom Rücken 2 weg. Dies liegt in der Biegung des Stützelements 5 an seinem oberen Ende 26 begründet, aber auch darin, daß die Wirbelsäule 16 in ihrer Normalstellung an dieser Stelle leicht vorwärts gebogen ist.

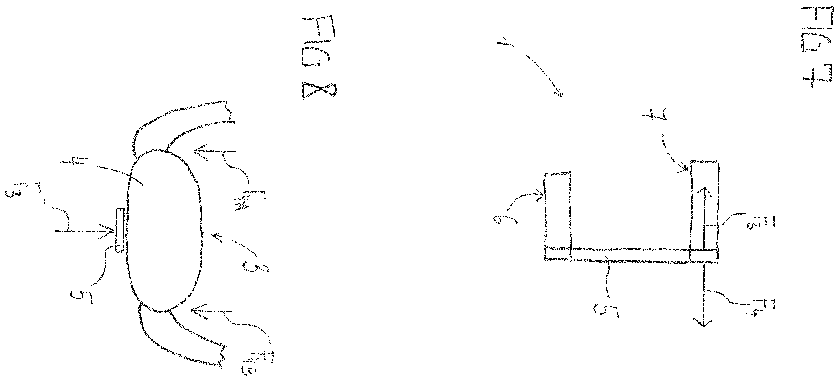

Bei dem Schließen des Brustgurtes 7 wird das obere Ende 26 des Stützelements 5 nach vorn gezogen und damit vorgespannt, siehe Fig. 7. Dabei wird der Beugeabschnitt 25 und mit diesem das Stützelement 5 elastisch verformt, insbesondere gerade gebogen. In einem solchen vorgespannten Zustand nimmt der die Rückenorthese 1 tragende Patient 3 eine aufrechte Körperhaltung ein. Bei dieser Körperhaltung befinden sich das obere Ende 26 des Stützelements 5 und das untere Ende 8 des Stützelements 26 entweder weitgehend vertikal übereinander, zumindest ist jedoch der Horizontalabstand zwischen dem oberen Ende 26 des Stützelements 5 und dem unteren Ende 8 des Stützelements 26 geringer als in einem nichtvorgespannten Zustand, siehe Fig. 6.

In jedem Punkt des Stützelements 5 entlang seiner Längserstreckung 15 wirkt im vorgespannten Zustand ein Moment einer bestimmten Größe gegen die Verbiegung des Stützelements 5. Der Brustgurt 7 wird mit anderen Worten gegen die Auslenkung des Stützelements 5 geschlossen. Die Zugkraft am Brustgurt 7 muß größer sein als die Gegenkraft, damit es zu einer Verformung des Stützelements 5 kommt. Hierdurch wird Energie (Federenergie) in dem Stützelement 5 gespeichert. Die in dem als Feder wirkenden Stützelement 5 gespeicherte potentielle Energie verursacht zum einen ein Zurückziehen der Wirbelsäule 16 nach hinten. Diese Zugwirkung auf die Wirbelsäule 16 resultiert in einer geänderten Haltung, genauer gesagt einer vorteilhaft veränderte Position der Wirbelsäule 16. Diese Zugwirkung wirkt dauerhaft, also kontinuierlich auf die Wirbelsäule 16 und bewirkt somit auch eine dauerhafte Haltungskorrektur. Mit Hilfe dieser ersten Unterstützung der Wirbelsäule 16 kann eine Haltungskorrektur um ca. 30 bis 40 mm erzielt werden. Der Patient 3 muß sich dabei nach dem Anlegen der Orthese 1 nur noch passiv verhalten. Es ist keine aktive Mitwirkung des Patienten 3 mehr erforderlich. Es ist mit anderen Worten nicht mehr erforderlich, daß sich der Patient 3 "daran erinnert", eine bessere Haltung einzunehmen. Dies erfolgt vielmehr zwangsläufig, automatisch und dauerhaft. Bei angelegter Orthese 1 wirkt ein Kräftegleichgewicht, wobei die genaue Kräfteverteilung durch die Krümmung des Stützelements 5 in jeder Stelle seiner Längserstreckung 15 definiert wird. Mit anderen Worten kann durch die Art und Weise der Krümmung des Stützelements 5 die lokale Kraftwirkung und damit die (Zug) -Wirkung der Orthese 1 auf die Wirbelsäule 16 entlang ihrer Längserstreckung 15 bestimmt werden. Das Kräftegleichgewicht am oberen Ende 26 des Stützelements 5 ist in Fig. 7 durch die Pfeile F3 und F4 angedeutet.

Nach dem Anziehen der Orthese 1 konzentrieren sich die Kräfte auf den Brust/Schulter-Bereich des Patienten 3. Betrachtet man eine vertikale Ebene durch den Brustkorb, dann wirken dort drei Kräfte. Der Brustgurt 7 drückt vorn auf die Brust 30. Die durch die Schultergurte 29 auf die rechte und die linke Schulter 31 wirkenden Kräfte F4A, F4B werden kompensiert durch die von hinten auf die Wirbelsäule 16 bzw. auf den Rücken 2 wirkende Kraft F3 des Stützelements 5, siehe Fig. 8. Durch die Verwendung der Orthese 1 öffnet sich der Brustkorb, so daß sich die Stellung der Wirbelsäule 16 und damit Haltung des Oberkörpers 4 verbessert. Durch den auf diese Weise "geöffneten" Brustkorb ergeben sich eine Reihe von weiteren positiven Folgen, insbesondere wird das Atmen erleichtert, da sich die für das Atmen zuständigen Muskeln besser bewegen können. Zusammenfassend zieht die mittig bzw. zentral am Rücken 2 angeordnete Orthese 1 den Oberkörper 4 des Patienten 3 ein Stück nach hinten, woraus nach dem Prinzip von Kraft und Gegenkraft automatisch ein Öffnen der Schultern 31 resultiert.

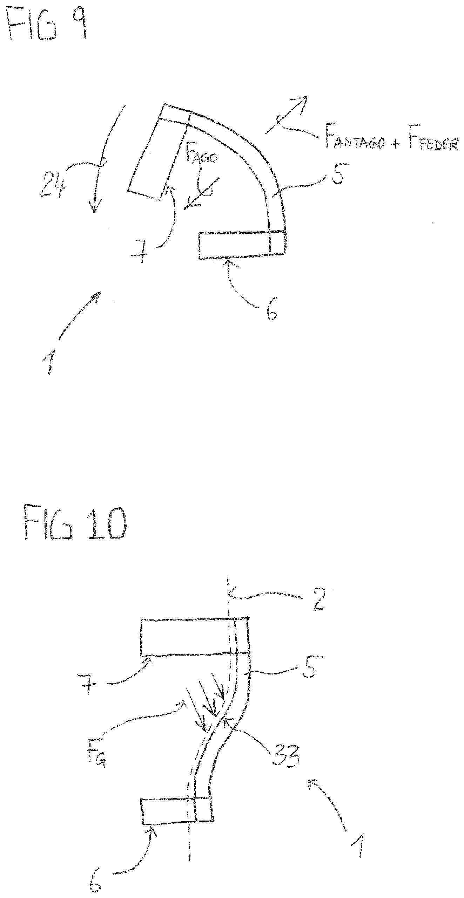

Nachfolgend wird die elastische Funktion des Stützelements 5 betrachtet, die zur Unterstützung antagonistischer Muskeln dient ("dynamische Betrachtung"), siehe Fig. 9.

Zum Zweck eines kraftunterstützten Aufrichtens eines nach vorn geneigten Oberkörpers 4 des Patienten 3 ist der obere Beugeabschnitt 25 bei einer nach vorn orientierten Neigung des Oberkörpers 4 über den Grad der Vorspannung hinaus weiter nach vorn spannbar derart, daß das Stützelement 5 ein auf den Oberkörper 4 wirkendes, entgegen der nach vorn orientierten Neigung rückstellendes Aufrichtmoment auf den Oberkörper 4 ausübt. Das bedeutet, daß die Orthese 1 nicht nur "statisch", z.B. im Stehen oder im Sitzen angewendet werden kann, sondern ihre Funktion auch in einer "dynamischen" Situation ausübt, d.h. in einer Neigebewegung 24 bzw. während einer solchen Neigebewegung 24 des Oberkörpers 4, wobei - wie weiter unten noch genauer erläutert - dabei eine Torsion des Oberkörpers 4 nicht behindert wird.

Bei einer Bewegung des Oberkörpers 4 nach vorn muß zusätzliche Arbeit verrichtet werden. Mit Hilfe der Muskeln wird das Stützelement 5 elastisch deformiert, nämlich nach vorn verbogen. Bei der Rückbewegung, also dem Aufrichten aus der nach vorn gebeugten Stellung versucht das Stützelement 5, das Energie gespeichert hat, den Oberkörper 4 in die aufrechte Position zurückzuziehen. Anders ausgedrückt hilft das Stützelement 5 bei dem Aufrichten des Oberkörpers 4. Die agonistischen Muskeln dienen zum Beugen nach unten, siehe Pfeil F (AGO) in Fig. 9. Diese Muskeln müssen etwas mehr Arbeiten als gewöhnlich, da sie zugleich das Stützelement 5 verformen, d.h. nach Art einer Feder spannen. Jedoch ist die Bewegung nach unten schwerkraftunterstützt und daher vergleichsweise einfach zu bewerkstelligen. Im umgekehrten Fall dienen die antagonistischen Muskeln zum Aufrichten des Oberkörpers 4 gegen die Schwerkraft, jetzt jedoch unterstützt durch die gespeicherte Federkraft in dem Stützelement 5, siehe Pfeil F(ANTAGO) + F(FEDER).

Zu Beginn des Beugevorgangs sind die wirkenden Kräfte im Gleichgewicht. Beim Beugen nach vorn bzw. unten ist die Kräftedifferenz nicht mehr Null, sondern resultiert in einer Bewegung. Der Patient 3 verrichtet Arbeit gegen eine Federkraft; das Stützelement 5 wird gespannt. Die kinetische Energie des Nach vorn-Beugens hat sich am Ende der Bewegung in potentielle Energie umgewandelt. Beim Aufrichten wird die potentielle Energie wieder in das Kräftesystem zurückgegeben. Die durch die antagonistischen Muskeln aufgebrachte Kraft wird durch die in die gleiche Richtung wirkende Federkraft unterstützt. Somit kann beim Aufrichten in die aufrechte Position von den antagonistischen Muskeln aufzubringende die Kraft kleiner sein.

Die Orthese 1 erfüllt nicht zuletzt auch eine Stützfunktion, siehe Fig. 10. In einer Vorwärtsbeugeposition werden die Antagonisten-Muskeln üblicherweise belastet und überanstrengt. Die Reaktion dieser Muskeln auf die Deformation des Stützelements 5 wird immer stärker, jedoch werden die Muskeln in der nach vorn gebeugter Position nicht überlastet, da das Gewicht des Oberkörpers 4 in erheblichem Maße von dem deformierten Stützelement 5 getragen wird. Hierzu weist das Stützelement 5 einen zwischen seinem oberen Ende 26 und seinem unteren Ende 8 angeordneten im wesentlichen biegesteifen Stützabschnitt 33 auf, der in der Trageposition zumindest zeitweise, nämlich in Abhängigkeit von der Neigung des Oberkörpers 4, den Rücken 2 abstützt, indem es einen Vertikalanteil der Gewichtskraft des Oberkörpers 4 des Patienten 3 aufnimmt, in Fig. 10 mit Pfeilen F(G) angedeutet. Speziell im Gleichgewichtszustand aber auch in einer Vorwärtsbeugeposition lastet ein Vertikalanteil des Gewichts des Oberkörpers 4 auf dem Stützelement 5. Auf diese Weise wird der Patient 3 beim Tragen seines Gewichts entlastet. In Abhängig von der Art und Weise der Krümmung des Stützelements 5 kann mehr oder weniger Gewicht des Patienten 3 von dem Stützelement 5 aufgenommen werden. Angriffspunkt der Kräfte ist dabei ein Mittelteil der Rückenstütze 5, nämlich der Stützabschnitt 33, der in der Trageposition eine horizontal verlaufende Komponente aufweist. Auf diesem Stützabschnitt 33 stützt sich die Wirbelsäule 16 bzw. der Rücken 2 des Patienten 3 ab.

Alle in der Beschreibung, den nachfolgenden Ansprüchen und den Zeichnungen dargestellten Merkmale können sowohl einzeln als auch in beliebiger Kombination miteinander erfindungswesentlich sein. Diese Merkmale bzw. Merkmalskombinationen können jeweils eine selbständige Erfindung begründen, deren Inanspruchnahme ausdrücklich vorbehalten ist.

### Bezugszeichenliste

- 1: Rückenorthese
- 2: Rücken
- 3: Patient
- 4: Oberkörper
- 5: Stützelement
- 6: Hüftgurt
- 7: Brustgurt
- 8: unteres Ende des Stützelements
- 9: erste Stelle, unterer Fixierpunkt
- 10: Verschluß
- 11: Verschluß
- 12: Aufnahmetasche
- 13: mittlerer Teil
- 14: zweite Stelle, oberer Fixierpunkt
- 15: Längserstreckung
- 16: Wirbelsäule
- 17: geschwungener Bereich
- 18: Länge
- 19: Breite
- 20: Dicke
- 21: erster S-förmiger Abschnitt
- 22: zweiter S-förmiger Abschnitt
- 23: Biegung
- 24: Neigebewegung
- 25: Beugeabschnitt
- 26: oberes Ende des Stützelements
- 27: Haken
- 28: Aufnahmeschlitz
- 29: Schultergurt
- 30: Brust
- 31: Schulter
- 32: Achsel
- 33: Stützabschnitt

## Patentansprüche

1. Rückenorthese (1) für den Rücken (2) eines Patienten (3), mit einem an einem Oberkörper (4) des Patienten (3) abnehmbar anbringbaren Stützelement (5),
mit einer Gurtanordnung (6, 7), die ausgebildet ist, in einer Trageposition der Rückenorthese (1) das Stützelement (5) körpernah an dem Rücken (2) des Patienten (3) zu fixieren,
wobei das Stützelement (5) strangartig ausgebildet ist und in der Trageposition eine Längserstreckung (15) im Wesentlichen entlang der Wirbelsäule (16) des Patienten (3) aufweist,
wobei das Stützelement (5) entlang seiner Längserstreckung (15) gekrümmt ist, nämlich eine Anzahl geschwungener Bereiche (17) aufweist derart, daß einer oder mehrere dieser geschwungenen Bereiche (17) an die menschliche Anatomie der Wirbelsäule (16) angepaßt sind,
wobei das Stützelement (5) einen oder mehrere elastisch flexible Beugeabschnitte (25) aufweist, welche Beugeabschnitte (25) eine reversible, zumindest abschnittsweise Beugung des Stützelements (5) quer zu seiner Längserstreckung (15) ermöglichen,
wobei die Gurtanordnung (6, 7) einen mit dem unteren Ende (8) des Stützelements (5) verbundenen Hüftgurt (6) umfaßt, der ausgebildet ist, in der Trageposition das Stützelement (5) an einer ersten Stelle (9) des Rückens (2) des Patienten (3) festzulegen, wobei sie den Oberkörper (4) des Patienten (3) vollumfänglich umschlingt,
wobei die Gurtanordnung (6, 7) einen mit einem mittleren Teil (13) und/oder einem oberen Teil des Stützelements (5) verbundenen Brustgurt (7) umfaßt, der ausgebildet ist, in der Trageposition das Stützelement (5) an einer zweiten Stelle (14) des Rückens (2) des Patienten (3) festzulegen, welche zweite Stelle (14) von der ersten Stelle (9) beabstandet ist,
**dadurch gekennzeichnet, daß** wenigstens einer der geschwungenen Bereiche (17) des Stützelements, nämlich ein an dem oberen Ende (26) des Stützelements (5) angeordneter oder das obere Ende (26) des Stützelements (5) bildender geschwungener Bereich (17), einen Beugeabschnitt (25) aufweist oder einen Beugeabschnitt (25) bildet, welcher Beugeabschnitt (25) in seinem nicht vorgespannten Zustand nicht an dem Rücken (2) des Patienten (3) anliegt, sondern von dem Rücken (2) absteht, und in der Trageposition durch die Gurtanordnung (6, 7) vorgespannt ist, zum Zweck einer dauerhaften Haltungskorrektur des Patienten (3) durch eine Zugwirkung auf die Wirbelsäule (16) nach hinten.

2. Rückenorthese (1) nach Anspruch 1, wobei der Hüftgurt (6) und/oder der Brustgurt (7) lösbar mit dem Stützelement (5) verbunden ist.

3. Rückenorthese (1) nach Anspruch 1 oder 2, wobei sich das Stützelement (5) in der Trageposition über einen wesentlichen Teil der Wirbelsäule (16) des Patienten (3) erstreckt.

4. Rückenorthese (1) nach einem der Ansprüche 1 bis 3, wobei das Stützelement (5) schienen- oder leistenförmig ausgeführt ist.

5. Rückenorthese (1) nach Anspruch 4, wobei das Stützelement (5) als flacher Profilstab mit Vierkant-Querschnitt ausgeführt ist.

6. Rückenorthese (1) nach einem der Ansprüche 1 bis 5, wobei das Stützelement (5) einteilig ausgeführt ist.

7. Rückenorthese (1) nach einem der Ansprüche 1 bis 6, wobei der Beugeabschnitt (25) in der Trageposition durch einen dann als Spanngurt wirkenden Teil des Brustgurtes (7) oder durch einen als Spanngurt wirkenden, mit dem Brustgurt (7) verbundenen Schultergurt (29) vorgespannt ist.

8. Rückenorthese (1) nach einem der Ansprüche 1 bis 7, wobei zum Zweck eines kraftunterstützten Aufrichtens eines nach vorn geneigten Oberkörpers (4) des Patienten (3) dieser Beugeabschnitt (25) bei einer nach vorn orientierten Neigung des Oberkörpers (4) über den Grad der Vorspannung hinaus weiter spannbar ist derart, daß das Stützelement (5) ein auf den Oberkörper (4) wirkendes, entgegen der nach vorn orientierten Neigung rückstellendes Aufrichtmoment auf den Oberkörper (4) ausübt.

9. Rückenorthese (1) nach einem der Ansprüche 1 bis 8, wobei das Stützelement (5) wenigstens einen Stützabschnitt (33) aufweist, welcher Stützabschnitt (33) in der Trageposition dauerhaft oder zumindest zeitweise, nämlich in Abhängigkeit von der Neigung des Oberkörpers (4), den Rücken (2) abstützt, indem es einen Vertikalanteil der Gewichtskraft des Oberkörpers (4) des Patienten (3) aufnimmt.

10. Rückenorthese (1) nach einem der Ansprüche 1 bis 9, wobei das Stützelement (5) torsionsweich ausgebildet ist.

## Claims

1. Back orthosis (1) for the back (2) of a patient (3),
with a support element (5) that can be removably attached to the upper body (4) of the patient (3),
with a belt arrangement (6, 7) which is designed, in a wearing position of the back orthosis (1), to fix the support element (5) close to the body on the back (2) of the patient (3),
wherein the support element (5) is designed in a strand-like manner and, in the wearing position, has a longitudinal extension (15) substantially along the spine (16) of the patient (3),
wherein the support element (5) is curved along its longitudinal extension (15), namely has a number of curved regions (17) such that one or more of these curved regions (17) are adapted to the human anatomy of the spine (16),
wherein the support element (5) has one or more elastically flexible bending sections (25), which bending sections (25) enable a reversible, at least section-wise bending of the support element (5) transversely to its longitudinal extension (15),
wherein the belt arrangement (6, 7) comprises a hip belt (6) connected to the lower end (8) of the support element (5), which is designed, in the wearing position, to fix the support element (5) at a first location (9) of the back (2) of the patient (3), wherein the hip belt (6) fully encircles the upper body (4) of the patient (3),
wherein the belt arrangement (6, 7) comprises a chest belt (7) connected to a middle part (13) and/or to an upper part of the support element (5), which is designed, in the wearing position, to fix the support element (5) at a second location (14) of the back (2) of the patient (3), which second location (14) is spaced apart from the first location (9),
**characterized in that** at least one of the curved regions (17) of the support element (5), namely a curved region (17) arranged at the upper end (26) of the support element (5) or forming the upper end (26) of the support element (5), has a bending section (25) or forms a bending section (25), which bending section (25), in its non-prestressed state, does not rest against the back (2) of the patient (3) but stands off from the back (2), and, in the wearing position, is prestressed by the belt arrangement (6, 7) for the purpose of a permanent posture correction of the patient (3) by means of a pulling action on the spine (16) rearward.

2. Back orthosis (1) according to claim 1, wherein the hip belt (6) and/or the chest belt (7) is releasably connected to the support element (5).

3. Back orthosis (1) according to claim 1 or 2, wherein the support element (5), in the wearing position, extends over a substantial part of the spine (16) of the patient (3).

4. Back orthosis (1) according to one of claims 1 to 3, wherein the support element (5) is designed in the form of a rail or strip-like element.

5. Back orthosis (1) according to claim 4, wherein the support element (5) is designed as a flat profile bar with a square cross-section.

6. Back orthosis (1) according to one of claims 1 to 5, wherein the support element (5) is designed in one piece.

7. Back orthosis (1) according to one of claims 1 to 6, wherein the bending section (25), in the wearing position, is prestressed by a part of the chest belt (7) acting as a tension belt or by a shoulder belt (29) acting as a tension belt and connected to the chest belt (7).

8. Back orthosis (1) according to one of claims 1 to 7, wherein, for the purpose of a force-assisted straightening of a forwardly inclined upper body (4) of the patient (3), the bending section (25), in the case of a forwardly oriented inclination of the upper body (4), is further tensionable beyond the degree of prestressing such that the support element (5) exerts on the upper body (4) a restoring straightening moment acting counter to the forwardly oriented inclination.

9. Back orthosis (1) according to one of claims 1 to 8, wherein the support element (5) has at least one support section (33), which support section (33), in the wearing position, permanently or at least temporarily, namely depending on the inclination of the upper body (4), supports the back (2) by absorbing a vertical component of the weight force of the upper body (4) of the patient (3).

10. Back orthosis (1) according to one of claims 1 to 9, wherein the support element (5) is designed to be torsionally compliant.

## Revendications

1. Orthèse dorsale (1) pour le dos (2) d'un patient (3), comprenant un élément de soutien (5) pouvant être fixé de manière amovible sur le torse (4) du patient (3),
avec un dispositif de sangles (6, 7) conçu pour fixer l'élément de soutien (5) près du corps, sur le dos (2) du patient (3), dans une position de port de l'orthèse dorsale (1),
l'élément de soutien (5) étant conçu sous forme de cordon et présentant, en position de port, une extension longitudinale (15) essentiellement le long de la colonne vertébrale (16) du patient (3),
l'élément de soutien (5) étant courbé le long de son extension longitudinale (15), c'est-à-dire qu'il présente un certain nombre de zones courbées (17) de telle sorte qu'une ou plusieurs de ces zones courbées (17) sont adaptées à l'anatomie humaine de la colonne vertébrale (16),
l'élément de soutien (5) comportant une ou plusieurs sections de flexion élastiquement flexibles (25), lesquelles sections de flexion (25) permettent une flexion réversible, au moins par sections, de l'élément de soutien (5) transversalement à son extension longitudinale (15),
l'agencement de sangles (6, 7) comprenant une ceinture abdominale (6) reliée à l'extrémité inférieure (8) de l'élément de soutien (5), qui est conçue pour fixer l'élément de soutien (5) en un premier endroit (9) du dos (2) du patient (3) en entourant complètement le torse (4) du patient (3),
l'agencement de sangles (6, 7) comprenant une sangle thoracique (7) reliée à une partie centrale (13) et/ou à une partie supérieure de l'élément de soutien (5), qui est conçue pour fixer l'élément de soutien (5) en position de portage à un deuxième emplacement (14) du dos (2) du patient (3), lequel deuxième emplacement (14) est espacé du premier emplacement (9),
**caractérisé en ce qu'**au moins l'une des zones courbes (17) de l'élément de soutien, à savoir une zone courbe disposée à l'extrémité supérieure (26) de l'élément de soutien (5) ou formant l'extrémité supérieure (26) de l'élément de soutien (5) (17) disposée à l'extrémité supérieure (26) de l'élément de support (5) ou formant l'extrémité supérieure (26) de l'élément de support (5), présente une partie courbée (25) ou forme une partie courbée (25), laquelle partie courbée (25), dans son état non précontraint, ne repose pas contre le dos (2) du patient (3), mais s'écarte du dos (2), et est précontrainte dans la position de portage par le dispositif à sangles (6, 7), dans le but d'une correction durable de la posture du patient (3) par une traction vers l'arrière sur la colonne vertébrale (16).

2. Orthèse dorsale (1) selon la revendication 1, dans laquelle la ceinture abdominale (6) et/ou la sangle thoracique (7) sont reliées de manière amovible à l'élément de soutien (5).

3. Orthèse dorsale (1) selon la revendication 1 ou 2, dans laquelle l'élément de soutien (5) s'étend, en position de portage, sur une partie importante de la colonne vertébrale (16) du patient (3).

4. Orthèse dorsale (1) selon l'une des revendications 1 à 3, dans laquelle l'élément de soutien (5) est réalisé sous forme d'attelle ou de barre.

5. Orthèse dorsale (1) selon la revendication 4, dans laquelle l'élément de soutien (5) est réalisé sous la forme d'une barre profilée plate à section carrée.

6. Orthèse dorsale (1) selon l'une des revendications 1 à 5, dans laquelle l'élément de soutien (5) est réalisé d'un seul tenant.

7. Orthèse dorsale (1) selon l'une des revendications 1 à 6, la partie de flexion (25) étant précontrainte en position de portage par une partie de la sangle thoracique (7) agissant alors comme une sangle de tension ou par une sangle d'épaule (29) reliée à la sangle thoracique (7) et agissant comme une sangle de tension.

8. Orthèse dorsale (1) selon l'une des revendications 1 à 7, dans laquelle, dans le but de redresser avec assistance mécanique le haut du corps (4) du patient (3) incliné vers l'avant, cette partie courbée (25) peut être tendue au-delà de l'inclinaison vers l'avant du haut du corps (4) incliné vers l'avant, au-delà du degré de précontrainte, de telle sorte que l'élément de soutien (5) exerce sur le torse (4) un moment de redressement agissant sur le torse (4) et s'opposant à l'inclinaison vers l'avant.

9. Orthèse dorsale (1) selon l'une des revendications 1 à 8, dans laquelle l'élément de soutien (5) comporte au moins une section de soutien (33), laquelle section de soutien (33) soutient le dos (2) en position de portage de manière permanente ou au moins temporaire, à savoir en fonction de l'inclinaison du haut du corps (4), en absorbant une partie verticale du poids du torse (4) du patient (3).

10. Orthèse dorsale (1) selon l'une des revendications 1 à 9, dans laquelle l'élément de soutien (5) est conçu pour être souple en torsion.
